# EUROPÄISCHE PATENTSCHRIFT

(11) **EP 4 185 346 B1**
(45) Veröffentlichungstag und Bekanntmachung des Hinweises auf die Patenterteilung: **02.09.2026**
(21) Anmeldenummer: 21754735.5
(22) Anmeldetag: 22.07.2021
(51) Int. Cl.: A61M 1/14, F04B 23/02, F04B 49/06, F04B 51/00, F04B 53/08

(54) **VERFAHREN ZUM ÜBERPRÜFEN DER FUNKTIONSFÄHIGKEIT VON FÖRDERMITTELN EINER MEDIZINISCHEN BEHANDLUNGSVORRICHTUNG, UND VORRICHTUNGEN**
METHOD FOR TESTING THE FUNCTIONALITY OF DELIVERY MEANS OF A MEDICAL TREATMENT DEVICE, AND DEVICES
PROCÉDÉ DE TEST DE LA FONCTIONNALITÉ DE MOYENS DE DISTRIBUTION D'UN DISPOSITIF DE TRAITEMENT MÉDICAL, ET DISPOSITIFS

(30) Priorität: 24.07.2020 DE 102020119654
(43) Veröffentlichungstag der Anmeldung: 31.05.2023
(73) Patentinhaber: Fresenius Medical Care Deutschland GmbH, 61352 Bad Homburg (DE)
(72) Erfinder: STOEHLEIN, Dieter, Herlheim 97509 (DE); SCHUELLER, Juergen, 97631 Bad Koenigshofen (DE); EHRENBERGER, Walter, 97447 Gerolzhofen (DE)
(74) Vertreter: Bobbert & Partner Patentanwälte PartmbB
(86) Internationale Anmeldenummer: PCT/EP2021/070529
(87) Internationale Veröffentlichungsnummer: WO 2022/018203

(56) Entgegenhaltungen:
- EP-B1- 1 364 194
- US-A- 5 263 367
- US-A1- 2012 312 726
- US-A1- 2019 209 766

## Beschreibung

Die vorliegende Erfindung betrifft ein Verfahren gemäß dem Oberbegriff des Anspruchs 1, eine Steuer- oder Regelvorrichtung gemäß dem Oberbegriff des Anspruchs 5 und eine medizinische Behandlungsvorrichtung gemäß dem Oberbegriff des Anspruchs 6. Des Weiteren betrifft die vorliegende Erfindung ein digitales Speichermedium gemäß Anspruch 14 und -ein Computerprogramm-Produkt gemäß Anspruch 15.

Es sind verschiedene Arten von medizinischen Behandlungsvorrichtungen bekannt. Zu den bekannten medizinischen Behandlungsvorrichtungen zählen beispielsweise Blutbehandlungsvorrichtungen, insbesondere die Vorrichtungen zur Hämodialyse, Hämofiltration und Hämodiafiltration. Während der extrakorporalen Blutbehandlung strömt das Blut in einem extrakorporalen Blutkreislauf durch eine Blutbehandlungseinheit. Bei den Vorrichtungen zur Hämodialyse, Hämofiltration und Hämodiafiltration ist die Blutbehandlungseinheit ein Dialysator oder Filter, der vereinfacht ausgedrückt durch eine semipermeable Membran in eine Blutkammer und eine Dialysierflüssigkeitskammer getrennt ist. Während der Blutbehandlung mittels Hämodialyse oder Hämodiafiltration strömt das Blut durch die Blutkammer, während eine Dialysierflüssigkeit durch die Dialysierflüssigkeitskammer strömt.

In manchen Blutbehandlungsvorrichtungen kann die Dialysierflüssigkeit durch volumetrische Mischung hergestellt werden. Unter volumetrischer Mischung ist zu verstehen, dass mindestens eine Flüssigkeit nach dem Volumen dosiert wird. Beispielsweise können Reinwasser und mindestens ein Flüssigkonzentrat volumetrisch dosiert und entsprechend einer vorgegebenen Rezeptur zu frischer Dialysierflüssigkeit vermischt werden.

Bei der volumetrischen Dosierung ist die Genauigkeit, mit der die verwendeten Fördermittel arbeiten, von entscheidender Bedeutung. Eine fehlerhafte Dosierung führt zu einer fehlerhaften Zusammensetzung der Dialysierflüssigkeit. Die Fördergenauigkeit eines Fördermittels kann sich mit der Zeit ändern. Ursachen hierfür können beispielsweise Undichtigkeiten, Quellungen von Werkstoffen, Ablagerungen im Inneren des Fördermittels und/oder den Leitungen und Verschleiß sein. Während Undichtigkeiten mit einem automatischen Integritätstest, beispielsweise einem Druckhaltetest, auf einfache Weise sicher erkannt werden können, sind andere Ursachen, die zu einer größeren oder einer kleineren Förderleistung führen können, nicht ohne weiteres automatisch erkennbar.

Um die Patientensicherheit nicht zu gefährden, ist eine fehlerhafte Dosierung auszuschließen. Deshalb werden die Fördermittel so robust ausgelegt, dass eine betriebsbedingte Änderung der Förderleistung während ihrer Lebensdauer möglichst klein ist. Dennoch kann sich die Förderleistung eines Fördermittels mit der Zeit ändern.

Die Förderleistung kann bei Verdacht auf Abweichungen, beispielsweise mittels Ausliterns, überprüft werden. Bei ungenügender Förderleistung wird das Fördermittel ausgetauscht. Solche Arbeiten werden in der Regel von einem qualifizierten Servicetechniker ausgeführt.

Je öfter die Fördermittel der medizinischen Behandlungsvorrichtung überprüft werden, desto zuverlässiger kann die Behandlungsvorrichtung arbeiten. Es besteht der Bedarf nach einer regelmäßigen automatischen Überprüfung der Förderleistung. Es besteht ein Bedarf nach einer automatischen Erkennung von Änderungen der Förderleistung eines Fördermittels, beispielsweise im Rahmen eines regelmäßigen automatischen Funktionstests.

Aus US 2019/209766 A1 ist ein Verfahren zum Überprüfen eines Flüssigkeitssystems einer medizinischen Blutbehandlungsvorrichtung bekannt.

Es ist Aufgabe der vorliegenden Erfindung, ein Verfahren zum Überprüfen der Fördergenauigkeit von Fördermitteln einer medizinischen Behandlungsvorrichtung vorzuschlagen. Ferner soll eine Steuer- oder Regelvorrichtung (kurz: Steuervorrichtung, die optional auch regeln kann) vorgeschlagen werden, mit der das Verfahren bewirkt oder veranlasst werden kann. Außerdem sollen weitere zum Durchführen des Verfahrens geeignete Vorrichtungen, insbesondere eine medizinische Behandlungsvorrichtung (kurz: Behandlungsvorrichtung), angegeben werden.

Die erfindungsgemäße Aufgabe wird durch das Verfahren mit den Merkmalen des Anspruchs 1 gelöst. Sie wird ferner gelöst mittels der Steuer- oder Regelvorrichtung mit den Merkmalen des Anspruchs 5, der medizinischen Behandlungsvorrichtung mit den Merkmalen des Anspruchs 6, des digitalen Speichermediums mit den Merkmalen des Anspruchs 14 und des Computerprogramm-Produkts mit den Merkmalen des Anspruchs 15.

Erfindungsgemäß wird somit ein Verfahren zum Überprüfen der Funktionsfähigkeit eines Fördermittels einer medizinischen Behandlungsvorrichtung zur extrakorporalen Blutbehandlung vorgeschlagen.

Das Verfahren umfasst vorzugsweise ein Bereitstellen eines vorzugsweise vollständig mit Flüssigkeit befüllten Behälters. Luft oder ein anderes Gas ist vorzugweise nicht im Behälter vorgesehen. Der Behälter steht in Fluidverbindung oder Förderverbindung mit dem Fördermittel.

Ferner umfasst das Verfahren ein Einstellen eines vorbestimmten, vorzugsweise eines aus einer Speichervorrichtung ausgelesenen und/oder in einer solchen dauerhaft gespeicherten, Anfangsdrucks im Behälter. Das Einstellen kann, rein exemplarisch, mittels Pumpen und/oder Ventilen in Leitungen, die den Behälter fluidisch mit einem Äußeren des Behälters verbinden, erfolgen. Beispielsweise kann mittels einer Füllpumpe der Behälter initial luftfrei gefüllt werden und mit der Ultrafiltrationspumpe für einen vorbestimmten Flüssigkeitsdruck im Inneren des Behälters gesorgt werden. Vorzugsweise wird der Anfangsdruck von dem auf seine Funktionsfähigkeit hin zu überprüfenden Fördermittel bewirkt, vorzugsweise nicht von einem anderen, ebenfalls auf seine Funktionsfähigkeit hin zu überprüfenden weiteren Fördermittel. Hierbei kann von einem definierten Füllen gesprochen werden, d. h. der nach Befüllen herrschende Druck ist bekannt und/oder vorbestimmt. Es liegt vorzugweise eine Differenz zum Atmosphärendruck vor.

Das Verfahren umfasst ein Ansteuern des Fördermittels zum Durchführen einer vorbestimmten bzw. festgelegten Anzahl an Förderbewegungen. Solche Förderbewegungen können Hübe, Rotationen, Schläge, Winkelschritte, Schritte usw. des Fördermittels oder seiner Bauteile sein, je nach Bauart des Fördermittels. Das Ansteuern kann also beispielsweise als ein Abrufen oder ein Anfordern der vorbestimmten Förderbewegungen verstanden werden. Diese Förderbewegungen beginnen vorzugsweise erst, nachdem der vorbestimmte Ausgangsdruck eingestellt worden ist. Die vorbestimmte oder festgelegte Anzahl an Förderbewegungen beginnt mit der ersten dieser Förderbewegungen vorzugsweise erst, nachdem für den Anfangsdruck gesorgt wurde bzw. dieser erreicht ist.

Weiter umfasst das Verfahren ein Messen des im Behälter herrschenden Drucks mittels einer Druckmessung, nachdem die vorbestimmte Anzahl an Förderbewegungen des Fördermittels abgeschlossen ist. Auf diese Druckmessung folgt ein Festlegen oder Bezeichnen des gemessenen bzw. ermittelten Drucks als Enddruck, der dem betrachteten Fördermittel zugeordnet werden kann.

Das Verfahren umfasst weiter ein Auslesen eines gespeicherten Referenzwerts aus einer ersten Speichervorrichtung. Der Referenzwert kann ein Referenzdruck oder eine Referenzdruckdifferenz sein. Der Referenzwert kann insbesondere +/-5 hPa bis +/-500 hPa gegenüber dem Atmosphärendruck betragen. Die erste Speichervorrichtung kann als Teil der medizinischen Behandlungsvorrichtung, beispielweise als deren interner Speicher, oder aber getrennt von dieser vorgesehen sein.

Das Verfahren umfasst weiter ein Ermitteln einer Druckdifferenz zwischen gemessenem Enddruck und gespeichertem Referenzwert. Alternativ wird eine Druckdifferenz ermittelt zwischen einer Differenz zwischen Anfangsdruck und Enddruck einerseits und dem gespeicherten Referenzwert andererseits.

Ferner umfasst das Verfahren den Schritt eines Auswertens der ermittelten Druckdifferenz, **z. B.** anhand eines vorbestimmten Kriteriums.

Ergebnis oder Ziel des Auswertens kann es sein, eine unzulässige Druckdifferenz zwischen dem Referenzwert, der dem Fördermittel zugeordnet ist und dem gemessenen Enddruck bzw. der ermittelten Druckdifferenz festzustellen.

Eine Druckmessung zu Beginn des Verfahrens kann unterbleiben, falls nach dem vorstehend beschriebenen Befüllen des Behälters mit ausreichender Genauigkeit davon ausgegangen werden darf, dass die Höhe des nach dem Befüllen im Behälter herrschenden Drucks bereits, etwa aufgrund des Befüllvorgangs als solchem, bekannt ist. Es kann in manchen Ausführungsformen vorgesehen sein, den herrschenden Druck auch bereits vor Ausführen der Förderbewegungen wenigstens einmal, beispielsweise zu Kontrollzwecken, zu messen.

Optional werden die oben beschriebenen Schritte in oben genannter Reihenfolge durchgeführt.

Erfindungsgemäß wird ferner eine Steuer- oder Regelvorrichtung, hierin auch kurz als Steuervorrichtung bezeichnet, vorgeschlagen. Sie ist konfiguriert, um im Zusammenwirken mit einer medizinischen Behandlungsvorrichtung das erfindungsgemäße Verfahren zu veranlassen oder durchzuführen.

Weiter wird erfindungsgemäß eine medizinische Behandlungsvorrichtung (kurz: Behandlungsvorrichtung) vorgeschlagen. Die erfindungsgemäße Behandlungsvorrichtung weist ein Flüssigkeitssystem, insbesondere ein Dialysierflüssigkeitssystem, mit einem Behälter für Flüssigkeit auf, eine Druckmesseinrichtung (vorzugsweise eine Absolutdruckmesseinrichtung) zum Messen eines im Behälter herrschenden Drucks sowie eine erste Speichervorrichtung, in welcher ein Referenzwert gespeichert ist, auf oder ist mit solchen Vorrichtungen verbunden. Ein Referenzwert ist hierin insbesondere ein Referenzdruck oder eine Referenzdruckdifferenz.

Die erfindungsgemäße Behandlungsvorrichtung weist ferner eine Auslesevorrichtung auf oder ist mit einer solchen verbunden. Die Auslesevorrichtung ist konfiguriert zum Auslesen des gespeicherten Referenzwerts, der dem Fördermittel zugeordnet ist, aus der ersten Speichervorrichtung.

Außerdem weist die erfindungsgemäße Behandlungsvorrichtung eine Ermittlungsvorrichtung auf oder ist mit einer solchen Vorrichtung verbunden. Die Ermittlungsvorrichtung ist konfiguriert zum Ermitteln einer Druckdifferenz zwischen gemessenem Enddruck und gespeichertem Referenzwert, oder zum Ermitteln einer Druckdifferenz zwischen einer Differenz zwischen Anfangsdruck und Enddruck einerseits und einer gespeicherten Druckdifferenz als gespeichertem Referenzwert andererseits.

Eine Auswertevorrichtung, welche zum Auswerten der ermittelten Druckdifferenz konfiguriert ist, ist ebenfalls von der erfindungsgemäßen Behandlungsvorrichtung umfasst oder mit dieser in Signalverbindung verbunden. Hierbei kann das Auswerten der ermittelten Druckdifferenz **z. B.** anhand eines vorbestimmten Kriteriums erfolgen.

Die Behandlungsvorrichtung umfasst wenigstens ein Fördermittel, welches mit dem Behälter und insbesondere seinem Inhalt oder Lumen in Fluidverbindung oder Förderverbindung steht, oder sie ist mit einem solchen Fördermittel verbunden.

Eine Steuer- oder Regelvorrichtung, insbesondere eine erfindungsgemäße Steuer- oder Regelvorrichtung ist ebenfalls von der erfindungsgemäßen medizinischen Behandlungsvorrichtung umfasst oder mit dieser verbunden. Die Steuer- oder Regelvorrichtung ist konfiguriert durch Zusammenwirken mit weiteren Einrichtungen oder Vorrichtungen der medizinischen Behandlungsvorrichtung das erfindungsgemäße Verfahren - insbesondere automatisch - zu veranlassen, durchzuführen, zu steuern und/oder zu regeln, insbesondere wie hierin offenbart.

Ein Zusammenwirken kann ein Ansteuern, Steuern oder Regeln sein oder umfassen. Ein Zusammenwirken kann eine Signalverbindung sein oder erfordern.

Die erfindungsgemäße medizinische Behandlungsvorrichtung kann für jeden der hierin genannten Schritte des erfindungsgemäßen Verfahrens eine entsprechend geeignete und/oder konfigurierte Einrichtung oder Vorrichtung wie z. B. eine Auswerteeinheit für die Auswertung, eine Druckmesseinrichtung für die Druckmessung, eine Befülleinrichtung für das Befüllen des Behälters (z. B. eine Pumpe, ein Ventil oder dergleichen) usw. aufweisen oder mit solchen Einrichtungen oder Vorrichtungen verbunden sein.

Ein erfindungsgemäßes, insbesondere digitales, insbesondere nicht-flüchtiges, Speichermedium (hier auch als Träger bezeichnet), insbesondere in Form von Diskette, RAM, ROM, CD, Festplatte, DVD, USB-Stick, Flashcard, SD-Karte oder EPROM, insbesondere mit elektronisch oder optisch auslesbaren Steuersignalen, kann derart konfiguriert sein, um eine Steuervorrichtung zu einer Steuervorrichtung zu konfigurieren, mit welcher das hierin beschriebene erfindungsgemäße Verfahren bewirkt werden kann. Alternativ oder ergänzend kann das digitale Speichermedium konfiguriert sein, um eine medizinische Behandlungsvorrichtung zu einer erfindungsgemäßen medizinischen Behandlungsvorrichtung zu konfigurieren, mit welcher das hierin beschriebene erfindungsgemäße Verfahren bewirkt oder ausgeführt werden kann.

Dabei können alle, einige oder manche der maschinell durchführbaren Schritte dieses Verfahrens veranlasst werden.

Ein erfindungsgemäßes Computerprogramm-Produkt weist einen volatilen, flüchtigen oder auf einem maschinenlesbaren Träger gespeicherten Programmcode auf, durch den eine Steuervorrichtung derart konfiguriert wird, dass das hierin beschriebene erfindungsgemäße Verfahren bewirkt werden kann. Alternativ oder ergänzend kann mittels des Computerprogramm-Produkts eine medizinische Behandlungsvorrichtung derart konfiguriert werden, dass das hierin beschriebene erfindungsgemäße Verfahren bewirkt oder ausgeführt werden kann.

Dabei können wiederum alle, einige oder manche der maschinell durchführbaren Schritte dieses Verfahrens veranlasst werden. Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte, ein EPROM und dergleichen sein.

Ein offenbarungsgemäßes Computerprogramm weist einen Programmcode auf, durch den eine Steuer- oder Regelvorrichtung oder eine medizinische Behandlungsvorrichtung derart konfiguriert wird, dass das hierin beschriebene erfindungsgemäße Verfahren bewirkt oder ausgeführt werden kann.

Dabei können alle, einige oder manche der maschinell durchführbaren Schritte dieses Verfahrens veranlasst werden.

Unter einem Computerprogramm-Produkt kann beispielsweise ein auf einem Träger gespeichertes Computerprogramm, ein Embedded System als umfassendes System mit einem Computerprogramm (z. B. ein elektronisches Gerät mit einem Computerprogramm), ein Netzwerk von computerimplementierten Computerprogrammen (z. B. Client/Serversystem, Cloud Computing System, etc.) oder ein Computer, auf dem ein Computerprogramm geladen ist, abläuft, gespeichert ist, ausgeführt oder entwickelt wird, verstanden werden.

Unter einem Computerprogramm kann beispielsweise ein physikalisches, vertriebsfähiges Software-Produkt verstanden werden, welches ein Programm aufweist. Bei allen vorstehenden und folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von "programmiert" oder "konfiguriert" die Rede ist, so ist auch offenbart, diese Begriffe gegeneinander auszutauschen.

Vorteilhafte Weiterentwicklungen der vorliegenden Erfindung sind jeweils Gegenstand von Unteransprüchen und Ausführungsformen.

Wenn hierin von einer Ausführungsform die Rede ist, so stellt diese eine erfindungsgemäße, beispielhafte Ausführungsform dar.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, z. B. im Sinne eines Disclaimers. Für jede hierin genannte Ausführungsform gilt somit, dass die gegenteilige Ausführungsform, beispielsweise als Negation formuliert, ebenfalls offenbart ist.

Erfindungsgemäße Ausführungsformen können eines oder mehrere der oben und/oder im Folgenden genannten Merkmale in jeder technisch möglichen Kombination aufweisen.

Der Anfangsdruck ist vorzugsweise jener Druck, von welchem ausgehend das auf seine Funktionsfähigkeit zu überprüfende Fördermittel zu fördern beginnt.

Der Referenzwert kann z. B. vom Hersteller etwa bei der Produktion oder vom Techniker bei der Inbetriebnahme in der ersten Speichervorrichtung und/oder vor Beginn des Betriebs des zu überprüfenden Fördermittels zum Zwecke seiner Überprüfung oder zum Durchführen des erfindungsgemäßen Verfahrens hinterlegt werden oder hinterlegt worden sein, welche wiederum Teil des Fördermittels oder der medizinischen Behandlungsvorrichtung, welche das Fördermittel aufweist, sein kann. Aus ihr kann der unveränderliche Referenzwert bei Ausführen des erfindungsgemäßen Verfahrens ausgelesen werden.

Der Referenzwert ist vorzugsweise nicht der Anfangsdruck und/oder kein Druck, der zum Überprüfen der Funktionsfähigkeit eines anderen Fördermittels mittels dieses anderen Fördermittels erzeugt wurde.

Sollte das Fördermittel zu einem späteren Zeitpunkt ausgetauscht werden, etwa aus Gründen der Wartung oder Reparatur, so kann, ähnlich wie bereits zum Zeitpunkt der Produktion oder Inbetriebnahme der Referenzwert neu ermittelt und - vorzugsweise netzausfallsicher - an geeigneter Stelle wie in der ersten Speichervorrichtung gespeichert werden, was ebenfalls Teil des erfindungsgemäßen Verfahrens sein kann.

Zur Vermeidung von Datenverlust (z.B. bei Austausch einzelner Rechnerplatinen) können Kopien des gespeicherten Referenzwertes auf mehrere Rechnerplatinen verteilt werden.

Referenzwerte können beispielsweise in einem Non-Volatile Random Access Memory (NOVRAM) hinterlegt sein; ein NOVRAM ist ein Speicherbaustein, der prinzipbedingt zyklisch beschrieben werden darf, bei dem aber trotzdem, bei auftretendem, externem Stromausfall, der letztgültige Dateninhalt im internen ROM-Bereich gesichert wird. Ein in einen NOVRAM integrierter Kondensator liefert hierbei die Energie, die nötig ist, um bei Ausfall der extern angelegten Spannung den Inhalt des, jeweils ebenfalls im NOVRAM integrierten, RAM-Bereichs in den ROM-Bereich umzukopieren.

Das erfindungsgemäße Verfahren umfasst ferner den Schritt des Einstellens einer vorbestimmten Temperatur (eines vorbestimmten Temperaturwerts) innerhalb des Behälters, um einen vorbestimmten bzw. festgelegten und damit vergleichbaren Zustand oder ebensolche Rahmenbedingungen für das Überprüfen, insbesondere die Druckmessung, zu erreichen.

Alternativ wird erfindungsgemäß die während des Ausführens des Verfahrens im Behälter herrschende Temperatur gemessen ohne zwingend auf die vorbestimmte Temperatur eingestellt zu werden. Die gemessene Temperatur kann in diesen Ausführungsformen optional berücksichtigt werden, indem z. B. Korrekturfaktoren den gemessenen Druck betreffend oder Umrechnungen des gemessenen Druckwerts in Abhängigkeit der gemessenen Temperatur erfolgen. Der Referenzwert kann in Abhängigkeit von der während der Überprüfung des Fördermittels herrschenden Temperatur z. B. in Tabellenform gespeichert und abgerufen werden.

In manchen Ausführungsformen des Verfahrens umfasst das Auswerten ein Ermitteln, ob die ermittelte Druckdifferenz oder ihr Betrag innerhalb vorbestimmter Grenzen liegt, einen Grenzwert über- oder unterschreitet, einen Mindestwert überschreitet und/oder einen Höchstwert nicht überschreitet, oder das Auswerten besteht hieraus. Hierdurch erfolgt ein Auswerten anhand eines Kriteriums (Grenzwert, Bereich, Höchstwert, usw.).

In einigen Ausführungsformen umfasst das Verfahren den zusätzlichen Schritt eines Ausgebens eines akustischen und/oder optischen Alarms. Insbesondere wird dieser Alarm ausgegeben, sollte das Auswerten zu vorab als unzulässig definierten Ergebnissen führen. Diese als unzulässige definierten Ergebnisse können beispielsweise in einer Speichervorrichtung hinterlegt sein. Die Speichervorrichtung kann der ersten Speichervorrichtung entsprechen oder eine andere Speichervorrichtung sein.

Alternativ oder ergänzend wird dieser Alarm insbesondere bei Verlassen vorbestimmter Grenzen, insbesondere bei Unterschreiten des unteren Grenzwerts oder Überschreiten des oberen Grenzwerts, bei Verlassen eines zulässigen Wertebereichs oder bei Übersteigen eines vorgegebenen Betrags der Druckdifferenz erfolgen.

In einer weiteren alternativen erfindungsgemäßen Ausführungsform umfasst das Verfahren den zusätzlichen Schritt des Speicherns des Enddrucks oder der ermittelten Druckdifferenz in einer zweiten Speichervorrichtung. Die zweite Speichervorrichtung kann z. B. eine interne Speichervorrichtung der medizinischen Behandlungsvorrichtung sein. Sie kann identisch zur ersten Speichervorrichtung sein oder unterschiedlich.

Das Verfahren umfasst den zusätzlichen Schritt des Ermittelns, z. B. des Errechnens, einer Verschleißkurve aus den gespeicherten Werten.

Eine solche Kurve kann ausgewertet werden, etwa um anhand ihrer Steigung oder anderen Eigenschaften frühzeitig eine Entwicklung oder einen Trend ausmachen zu können.

In manchen Ausführungsformen weist der Behälter Abschnitte des Altwasserzweigs eines Bilanziersystems auf oder besteht hieraus.

In einigen Ausführungsformen ist der Behälter ein Abschnitt in einer Flüssigkeitsleitung eines Dialysierflüssigkeitssystems einer medizinischen Behandlungsvorrichtung oder umfasst einen solchen.

In manchen Ausführungsformen ist das Fördermittel eine Ultrafiltrationspumpe, eine Bicarbonatpumpe oder eine Natriumpumpe.

In einigen Ausführungsformen ist wenigstens ein Fördermittel eine Verdrängerpumpe, insbesondere eine Membranpumpe, Exzenter-Membranpumpe, Schlauchpumpe, Rollenpumpe oder Kolbenpumpe.

In manchen Ausführungsformen ist wenigstens ein Fördermittel eine Strömungspumpe. Bei Strömungspumpen wird die Förderung ausschließlich durch strömungsmechanische Vorgänge bewirkt. Hierbei besteht eine ständige Verbindung zwischen der Saug- und der Druckseite der Pumpe, d. h. das Medium durchströmt die Maschine frei, also ohne Klappen und Ventile. Im Stillstand könnte das Medium die Pumpe rückwärts, also gegen die Pumprichtung, durchströmen. Zu diesem Typ zählen z. B. Zahnradpumpen, Flügelradverdichter, Zentrifugalpumpen usw.

Eine volumengenaue Förderung ist in diesen Ausführungsformen nur mit zusätzlichem Aufwand möglich, beispielsweise mittels Flusspumpe und Bilanzkammer. In manchen Ausführungsformen, in welchen Pumpen dieses Typs angewendet wird, wird vorzugsweise eine definierte Förderrate (beispielsweise eine Drehzahl, oder eine Drehzahl pro Zeiteinheit) eingestellt, mit welcher Flüssigkeit in das System oder den Behälter gedrückt oder aus diesem herausgesaugt wird, da bei diesem Pumpentyp kein definiertes Volumen entzogen wird. Der Verschleiß kann anhand des individuell mittels des Fördermittels erzielten Über\- oder Unterdrucks erkannt werden.

In manchen Ausführungsformen umfasst die medizinische Behandlungsvorrichtung eine Heizvorrichtung, konfiguriert zum Aufheizen der im Behälter vorliegenden Flüssigkeit auf eine vorbestimmte Temperatur zum Zwecke des Erreichens eines vorbestimmten bzw. festgelegten Zustands oder von Rahmenbedingungen während der auszuführenden Druckmessung. Entsprechende Sensoren für eine Feedbackschleife können vorgesehen sein.

Die Behandlungsvorrichtung umfasst ferner in einigen Ausführungsformen eine Vorrichtung, welche konfiguriert ist zum Ermitteln, ob die ermittelte Druckdifferenz innerhalb vorbestimmter Grenzen liegt, einen Grenzwert über- oder unterschreitet, einen Mindestwert überschreitet und/oder einen Höchstwert nicht überschreitet.

In manchen Ausführungsformen umfasst die Behandlungsvorrichtung eine Vorrichtung zum Ausgeben eines Alarms. Diese kann konfiguriert sein, um einen akustischen und/oder optischen Alarm auszugeben, sollte das Auswerten unzulässige Ergebnisse ergeben. Insbesondere bei Verlassen vorbestimmter Grenzen, bei Unterschreiten des unteren Grenzwerts oder Überschreiten des oberen Grenzwerts, bei Verlassen eines zulässigen Wertebereichs, bei Übersteigen eines vorgegebenen Betrags kann vorgesehen sein, einen solchen Alarm auszugeben.

In einigen Ausführungsformen umfasst die Behandlungsvorrichtung eine zweite Speichervorrichtung konfiguriert zum Speichern des Enddrucks oder der ermittelten Druckdifferenz in der zweiten Speichervorrichtung. Die zweite Speichervorrichtung kann z. B. eine interne Speichervorrichtung der medizinischen Behandlungsvorrichtung sein, sie kann identisch oder unterschiedlich zur ersten Speichervorrichtung sein.

Die zweite Speichervorrichtung kann Teil der medizinischen Behandlungsvorrichtung sein, beispielsweise in einem bei Wartungstätigkeiten regelmäßig ausgelesenen Bereich. Sie kann jedoch auch fern der medizinischen Behandlungsvorrichtung z. B. bei einer mit der Wartung oder Überwachung der Behandlungsvorrichtung betrauten Stelle angeordnet sein.

In manchen Ausführungsformen der medizinischen Behandlungsvorrichtung umfasst diese eine Vorrichtung, welche zum Ermitteln, **z. B.** Errechnen, einer Verschleißkurve aus den gespeicherten Werten konfiguriert ist.

Eine medizinische Behandlungsvorrichtung gemäß der vorliegenden Erfindung kann, ohne darauf beschränkt zu sein, zum Durchführen einer Blutbehandlung, insbesondere einer Hämodialyse, einer Hämofiltration, einer Hämodiafiltration oder einem Separationsverfahren geeignet und/oder konfiguriert sein.

Wird über eine Vielzahl von (mindestens zwei) Abläufen des erfindungsgemäßen Verfahrens zu verschiedenen Zeitpunkten hinweg eine schleichende oder zunehmende Verschlechterung der Fördergenauigkeit eines Fördermittels erkannt, so kann in einigen Ausführungsformen diesbezüglich ein Alarm oder eine Mitteilung ergehen.

So kann beispielsweise von der medizinischen Behandlungsvorrichtung eine Warnung oder ein Hinweis darauf ergehen, dass eine Wartung, Reparatur oder ein Austausch des Fördermittels wegen ungenügender Fördergenauigkeit ansteht. Auf diese Weise kann ein Austausch bereits beizeiten, d. h. vor seinem tatsächlich eintretenden, vollständigen Ausfall erfolgen, was wiederum vorteilhaft dazu beitragen kann, die Ausfallzeit der Behandlungsvorrichtung zu reduzieren. Eine Wartung oder Reparatur des als unzulänglich erkannten Fördermittels kann frühzeitig eingeplant werden und damit, anders als eine als Reaktion erfolgende Wartung, nicht erst zur Unzeit, d. h. zu einem evtl. unpassenden Zeitpunkt, erfolgen.

Wird das erfindungsgemäße Verfahren, wie in manchen Ausführungsformen angedacht, z. B. vor jeder Behandlungssitzung, bei der die Behandlungsvorrichtung eingesetzt wird, oder in beliebig engen Zeitabständen durchgeführt, so kann der Zeitpunkt, zu welchem eine Wartung oder Reparatur des unzulänglichen Fördermittels am besten in Angriff genommen werden sollte, vergleichsweise früh erkannt werden. Der Zeitpunkt, zu dem eine Störung des Betriebs der Behandlungsvorrichtung aufgrund des unzulänglichen Fördermittels eintreten wird, kann vorteilhaft mittels des erfindungsgemäßen Verfahrens unter Verwendung entsprechender Auswertung der erhobenen Messwerte oder Ergebnisse weitestgehend vorhersagbar werden.

In einigen Ausführungsformen werden die Ergebnisse des erfindungsgemäßen Verfahrens (als Auswertungsergebnisse, die Abweichung von einer tatsächlichen Fördertätigkeit, usw.), z. B. an der Behandlungsvorrichtung (Display) oder auf einem externen Monitor, Display oder dergleichen, dargestellt oder ausgedruckt.

In einigen Ausführungsformen werden die Ergebnisse oder zumindest die als relevant erkannten Ergebnisse des erfindungsgemäßen Verfahrens nach dessen Durchführung an der Behandlungsvorrichtung wiederaufrufbar in einem Datenspeicher gespeichert und/oder weiterverarbeitet. Dieser Datenspeicher kann Teil der medizinischen Behandlungsvorrichtung sein, oder getrennt von dieser vorliegen. Die Ergebnisse können weiterverarbeitet werden, um eine Verschleißkurve über der Zeit bezogen auf das Fördermittel zu erstellen. Diese kann bei Bedarf z. B. von einem Servicetechniker ausgelesen werden. Der Datenspeicher kann alternativ auf einem externen Gerät vorliegen. So können Ergebnisse z. B. in ein Netzwerk eingespeist und dort gespeichert werden. Gegebenenfalls können die Ergebnisse dann von mehreren Geräten aus abgerufen werden.

In manchen Ausführungsformen wird eine Wahrscheinlichkeit für das Auftreten einer Störung durch Fehlfunktion eines oder jedes Fördermittels nach bekannten Verfahren ermittelt und mitgeteilt, z. B. per Speicherung im auslesbaren Datenspeicher, per Display, Alarm, usw. Ergänzend oder alternativ hierzu kann der Zeitpunkt oder Zeitraum, zu dem bzw. in dem eine solche Störung vermutlich auftreten wird, ermittelt und mitgeteilt werden.

In einigen Ausführungsformen kommen zum Ermitteln u. a. der Wahrscheinlichkeit für das Auftreten einer Störung durch Fehlfunktion eines Fördermittels oder dessen Zeitpunkt oder Zeitraum bekannte Verfahren der Trendanalyse zum Einsatz.

Die zum Ausführen der vorgenannten Ermittlung von Wahrscheinlichkeit, Zeitpunkt/Zeitraum und/oder der Darstellung und/oder Übermittlung der sich hieraus ergebenden Ergebnisse erforderliche Hard- oder Software ist optional vorgesehen und entsprechend eingerichtet, konfiguriert und/oder programmiert.

Mittels Trendanalyse lassen sich Trends - also sich mittel- oder langfristig abzeichnende Entwicklungen - ermitteln und optional quantifizieren.

Bekannte Verfahren der Trendanalyse umfassen die einfache Mittelwertbildung, die Bestimmung gleitender Durchschnitte, das Ermitteln der kleinsten quadratischen Abweichung, das exponentielle Glätten erster Ordnung, usw.

In manchen Ausführungsformen weist der Behälter wenigstens einen Leitungsabschnitt auf. Alternativ weist er mehrere miteinander in Fluidkommunikation stehende Leitungsabschnitte auf, die vorzugsweise Teil der Behandlungsvorrichtung, insbesondere deren Dialysierflüssigkeitssystem, sind. Solche Leitungsabschnitte können derart miteinander in Fluidkommunikation stehen, dass sich ein in ihnen herrschender Fluidruck ungehindert innerhalb des Verbundes von Leitungen einstellen kann.

In einigen Ausführungsformen ist der Behälter ein Gefäß. Was hierin über den Behälter ausgeführt ist, gilt in diesen Ausführungsformen somit uneingeschränkt auch dessen Ausgestaltung als Gefäß.

In einigen Ausführungsformen ist die Flüssigkeit Dialysierflüssigkeit. In einigen bevorzugten Ausführungsformen ist die Flüssigkeit reines, entgastes Wasser ohne Zusätze (RO-Wasser, Reverse Osmosis-Wasser).

Alleine aufgrund des Auswertens der Messwerte der Druckmessung kann in einigen Ausführungsformen bestimmt werden, ob das Fördermittel ungenau fördert und welche Beträge die Abweichungen aufweisen. Ferner kann in anderen Ausführungsformen bestimmt werden, ob das Fördermittel in absehbarer Zukunft ungenau (oder im schlimmsten Fall gar nicht) fördern wird. Dann kann vorteilhafterweise ein rechtzeitiger Austausch des Fördermittels veranlasst werden.

Da mit der erfindungsgemäßen medizinischen Behandlungsvorrichtung das hierin beschriebene erfindungsgemäße Verfahren durchführbar und die Behandlungsvorrichtung mittels ihrer Steuervorrichtung zu seinem Ausführen konfiguriert ist, wird zur Vermeidung von Wiederholungen auf die vorstehend beschriebenen Ausführungen zu selbigem verwiesen.

In manchen Ausführungsformen fördert, während das erfindungsgemäße Verfahren abläuft oder als Schritt des Verfahrens, keines der drei Fördermittel in den Behälter hinein.

In einigen Ausführungsformen fördert keines der drei Fördermittel in den Behälter hinein, es sei denn, dies dient zum vollständigen Auffüllen des Behälters mit Flüssigkeit.

In manchen Ausführungsformen fördert keines der drei Fördermittel in den Behälter hinein, es sei denn, dies dient dem Einstellen des vorbestimmten Anfangsdrucks, bevor das in Frage stehende Fördermittel angesteuert wird zum Erbringen der zum Herausfördern des vorbestimmten Flüssigkeitsvolumens erforderlichen Soll-Fördertätigkeit.

In einigen Ausführungsformen fördert das Fördermittel im Rahmen des erfindungsgemäßen Verfahrens ausgehend von stets demselben Anfangsdruck. Ein Erhöhen des Drucks im Behälter mittels der Fördermittel ist in diesen Ausführungsformen nicht vorgesehen.

Der Behälter ist bei der Überprüfung des Fördermittels in manchen Ausführungsformen stets derselbe. Der in ihm herrschende Anfangsdruck kann stets derselbe sein, dies ist jedoch nicht zwingend der Fall.

In einigen Ausführungsformen werden Absolutwerte eines einzelnen Drucks betrachtet oder Differenzen zwischen Drücken.

Wenn hierin von einem Messen eines Drucks die Rede ist, so kann sich dieser Begriff in einigen Ausführungsformen auch auf das Ermitteln des gesuchten Drucks erstrecken, etwa indem der Druck aus vorliegenden Größen errechnet wird.

In manchen Ausführungsformen wird kein Volumen gemessen, verglichen und/oder ausgewertet.

In einigen Ausführungsformen wird bis einschließlich des Schritts des Auswertens der ermittelten Druckdifferenz nur die Funktion genau eines Fördermittels geprüft.

In manchen Ausführungsformen kommt bis einschließlich des Schritts des Auswertens der ermittelten Druckdifferenz nur genau ein Fördermittels zum Einsatz oder es fördert nur genau ein Fördermittel, insbesondere nur ein zu überprüfendes Fördermittel.

Wenn hierin von einer Signal- oder Kommunikationsverbindung zweier Komponenten die Rede ist, so kann hierunter eine im Gebrauch bestehende Verbindung zu verstehen sein. Ebenso kann hierunter zu verstehen sein, dass eine Vorbereitung zu einer solchen (kabelgebunden, kabellosen oder auf andere Weise umgesetzte) Signalkommunikation besteht, beispielsweise durch eine Kopplung beider Komponenten, etwa mittels *pairing,* usw.

Unter *pairing* versteht man einen Prozess, der im Zusammenhang mit Rechnernetzwerken erfolgt, um eine anfängliche Verknüpfung zwischen Rechnereinheiten zum Zwecke der Kommunikation herzustellen. Das bekannteste Beispiel hierfür ist das Herstellen einer Bluetooth-Verbindung, mittels welcher verschiedene Einrichtungen (z. B. Smartphone, Kopfhörer) miteinander verbunden werden. Pairing wird gelegentlich auch als *bonding* bezeichnet.

In manchen Ausführungsformen umfasst das erfindungsgemäße Verfahren keine Berechnung des im Behälter vorliegenden initialen Flüssigkeitsvolumens und/oder initialen Luftvolumens.

In einigen Ausführungsformen ist oder umfasst der Behälter keine Mischkammer.

In manchen Ausführungsformen weist die Behandlungsvorrichtung Rollen zu ihrer Fortbewegung auf. In einigen Ausführungsformen weist die Behandlungsvorrichtung einen Touchscreen zur Nutzung durch das medizinische Personal auf.

Mittels mancher erfindungsgemäßer Ausführungsformen können einer oder mehrere der hierin genannten Vorteile erzielbar sein, zu welchen folgende zählen:
Mittels des erfindungsgemäßen Verfahrens kann eine verlässliche Aussage über die Funktionstüchtigkeit und insbesondere über ihre Fördergenauigkeit des betrachteten Fördermittels getroffen werden, vorzugsweise durch Vergleich von gemessenen Druckwerten oder -differenzen mit gespeicherten Referenzwerten.

Ein weiterer Vorteil der Erfindung besteht darin, dass ein medizinisches Behandlungsgerät in die Lage versetzt wird, die Überprüfung auf ausreichende Genauigkeit der Fördertätigkeit seiner Fördermittel automatisch durchzuführen. Der Einsatz eines qualifizierten Servicetechnikers oder eines Anwenders ist hierzu nicht erforderlich oder kann auf gelegentliche Überprüfung beschränkt werden, was auch für Einsparung von Kosten beitragen kann.

Zudem können Abweichungen in der Fördertätigkeit eines Fördermittels, die zwischen Besuchen des qualifizierten Servicetechnikers oder Überprüfungen des Anwenders erstmals auftreten, frühzeitig, etwa im Rahmen einer täglichen Routineüberprüfung, erfasst werden.

Ein weiterer Vorteil ist die Verbesserung der Benutzerfreundlichkeit einer gattungsgemäßen Blutbehandlungsvorrichtung. Der Anwender wird erfindungsgemäß nicht mit der Überprüfung und Korrektur der Förderleistung von Fördermitteln belastet. Ausfallzeiten der Blutbehandlungsvorrichtung während des Einsatzes eines qualifizierten Servicetechnikers zur Überprüfung, etwa mittels Wiegens oder Einsatzes von Messzylindern, sind nicht oder seltener erforderlich.

Da das erfindungsgemäße Vorgehen auf einem definierten Befüllen (Druck, ggf. auch Temperatur) eines Behälters aufbaut, welcher in der Praxis aus anderen Gründen als der Überprüfung der Fördermittel ohnehin zu füllen sein wird, kann ein Teil der an sich erforderlichen Arbeit für die Implementierung des vorliegenden Verfahrens vorteilhaft entfallen: zumindest das erstmalige Befüllen dürfte in vielen praktischen Anwendungen bereits ohnehin erfolgt sein.

Das erfindungsgemäße Verfahren kann ermöglichen, die Fördervolumina der Pumpen ohne großen zusätzlichen Aufwand zu überprüfen. Durch die Speicherung der ermittelten Druckwerte und Druckdifferenzen und das Erstellen einer Verschleißkurve können Vorhersagen über einen in der näheren Zukunft möglichen Ausfall möglich sein. Somit könnte ein Servicetechniker bei der nächsten sicherheitstechnischen Kontrolle (STK) die zu erwartende Restlebensdauer der Pumpe abfragen und die Pumpe ggf. vorzeitig austauschen. Dies trägt vorteilhaft dazu bei, Service-Einsätze zu reduzieren und somit Kosten einzusparen.

Auch können mittels der vorliegenden Erfindung vorteilhaft eventuell aufgetretene Schäden, die den Pumpbetrieb beeinflussen können, z. B. abgelöste, abgerissene oder beschädigte Membranen beispielsweise bedingt durch Verschleiß, ermittelt werden, was insbesondere bei der Verwendung von Exzentermembranpumpen relevant ist. Die Anzahl aufwändiger Tests der Pumpen durch einen Servicetechniker kann somit vorteilhafterweise reduziert werden oder ganz entfallen.

Durch das frühzeitige Erkennen der Notwendigkeit eines Austausches des Fördermittels kann somit eine Erhöhung der Zuverlässigkeit der medizinischen Behandlungsvorrichtung liegen, denn je genauer die Fördermittel der Behandlungsvorrichtung fördern, desto sicherer für den Patienten kann diese arbeiten.

Weiterhin kann von Vorteil sein, dass mittels des erfindungsgemäßen Verfahrens die statistische Auswertung von Ausfallzahlen betreffend die betrachteten Fördermittel verbessert werden kann. Dies hilft vorteilhafterweise, echte Fehler zu ermitteln und die Dunkelziffer von funktionsuntüchtigen Fördermitteln zu reduzieren oder ganz wegfallen zu lassen.

Schließlich kann von Vorteil sein, dass mittels der vorliegenden Erfindung Fördermittel überprüft werden können, auch wenn in der medizinischen Behandlungsvorrichtung keine Mischkammer, insbesondere keine Mischkammer mit eigener Überwachungssensorik, die man zur Überprüfung der Fördermittel nutzen könnte, vorgesehen ist, wie dies in einigen Ausführungsformen der Fall sein kann.

Im Folgenden wird die Erfindung anhand bevorzugter Ausführungsformen derselben unter Bezugnahme auf die angehängte Zeichnung beschrieben. Das erfindungsgemäße Verfahren und die erfindungsgemäße Blutbehandlungsvorrichtung werden am Beispiel einer Hämodialysevorrichtung beschrieben. Das erfindungsgemäße Verfahren kann in gleicher Weise aber auch bei anderen Blutbehandlungsvorrichtungen, beispielsweise einer Hämodiafiltrationsvorrichtung, Verwendung finden. In den Figuren gilt:
- **Fig. 1**: zeigt Teile eines Leitungsdiagramms eines Flüssigkeitssystems einer erfindungsgemäßen medizinischen Behandlungsvorrichtung in einer ersten Ausführungsform;
- **Fig. 1a**: zeigt einen Ausschnitt A aus Fig. 1, welcher auf den in Fig. 1 gezeigten Behälter beschränkt ist;
- **Fig. 1b**: zeigt einen alternativen Behälter, der zur Überprüfung von bezogen auf die Fig. 1 alternativen Fördermitteln geeignet ist;
- **Fig. 2**: zeigt schematisch vereinfacht den Ablauf des erfindungsgemäßen Verfahrens anhand der hierfür verwendeten Vorrichtungen;
- **Fig. 3**: zeigt schematisch vereinfacht eine Exzenter-Membranpumpe als exemplarische Ausführungsform eines Fördermittels; und
- **Fig. 4**: zeigt einen exemplarischen Ablauf des erfindungsgemäßen Verfahrens in einem Druck-Förderbewegungs-Diagramm.

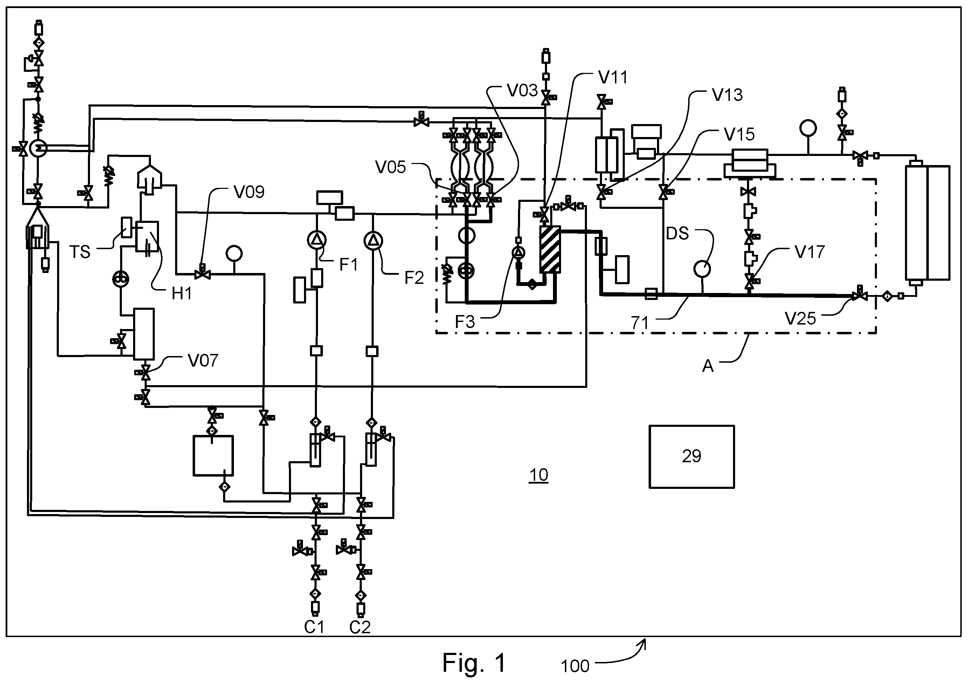

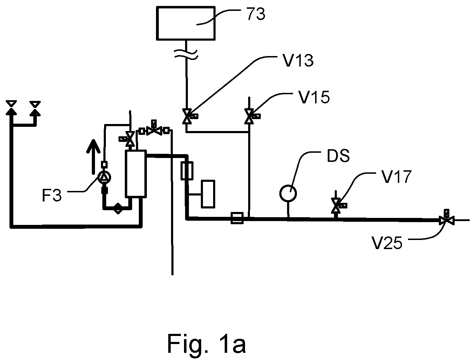

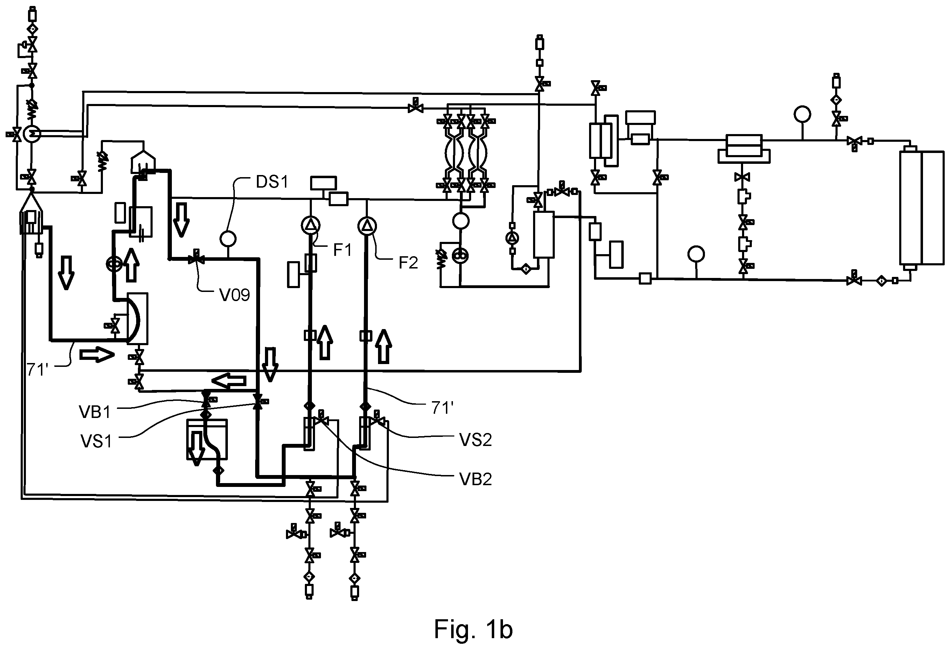

**Fig. 1** zeigt Teile eines Leitungsdiagramms eines Dialysierflüssigkeitssystems 10 (auch als Hydraulik bezeichnet) - als Beispiel eines Flüssigkeitssystems - einer erfindungsgemäßen medizinischen Behandlungsvorrichtung 100 in einer ersten Ausführungsform, hier rein optional einer Blutbehandlungsvorrichtung.

Das Dialysierflüssigkeitssystem 10 weist eine Vielzahl von Pumpen, Ventilen, Aktoren, Sensoren und weiteren Bauteilen auf. Sie alle können unabhängig voneinander mit einer Steuer- oder Regelvorrichtung 29 der medizinischen Behandlungsvorrichtung 100 in Signalverbindung stehen und optional von dieser angesteuert und/oder ausgelesen werden.

Zu den vorstehend genannten Pumpen zählen u. a. ein erstes Fördermittel F1, hier eine Bicarbonatpumpe, ein zweites Fördermittel F2, hier eine Natriumpumpe, sowie die hier mit Fördermittel F3 bezeichnete Ultrafiltrationspumpe.

Die Pumpen sind in Fluidkommunikation miteinander in das Dialysierflüssigkeitssystem 10 eingebunden. Das Fördermittel F3 ist mittels der in für die Fig. 1a extrahierten und hier und in Fig. 1a fett dargestellten Leitungen fluidisch verbunden mit einer Lüftungsvorrichtung sowie mit einem Temperatursensor TS als auch mit einem Drucksensor DS. Die sie verbindenden Leitungen sind einerseits durch das (stehende) Fördermittel F3, und andererseits durch die (geschlossenen) Ventile V03, V05, V11, V13, V15, V17 und V25 von einem Äußeren fluidisch getrennt. Das mittels des vorstehend genannten Fördermittels F3 und der Ventile V11, V13, V15, V17 und V25 begrenzte Volumen ergibt somit ein abgeschlossenes Gefäß oder einen Verbund von abgeschlossenen, aber miteinander kommunizierenden Leitungen, insbesondere Leitungsabschnitten, oder Gefäßen, und wird hierin als Behälter bezeichnet. Andere Bezeichnungen, etwa Volumen, Aufnahmeabschnitt für Flüssigkeit, usw. wären im Rahmen der vorliegenden Erfindung anstelle von "Behälter" ebenfalls vorstellbar.

Der Behälter, der in Fig. 1a unter Weglassen jener Elemente aus Fig. 1, die am Zustandekommen seines abgeschlossenen Volumens nicht beteiligt sind, gezeigt ist, wird hierin als Behälter 71 bezeichnet, siehe Fig. 1a.

Das Fördermittel F3 ist mit Bezug auf den Behälter 71 derart angeordnet, dass es die Flüssigkeit, die sich im Behälter 71 befindet, aus diesem herausfördern kann, was es bei Ablauf des erfindungsgemäßen Verfahrens in einer exemplarisch und nachfolgend beschriebenen Ausführungsform auch tut.

Der im Behälter angeordnete Druckmesser DS, mittels welchem z. B. die Druckmessung erfolgen kann, kann wie jeder andere verwendete Drucksensor optional die höchste Messgenauigkeit aller im Behälter messenden Druckmesser aufweisen. Der eingesetzte Druckmesser kann ein Analog-Digital-Converter (ADC) sein.

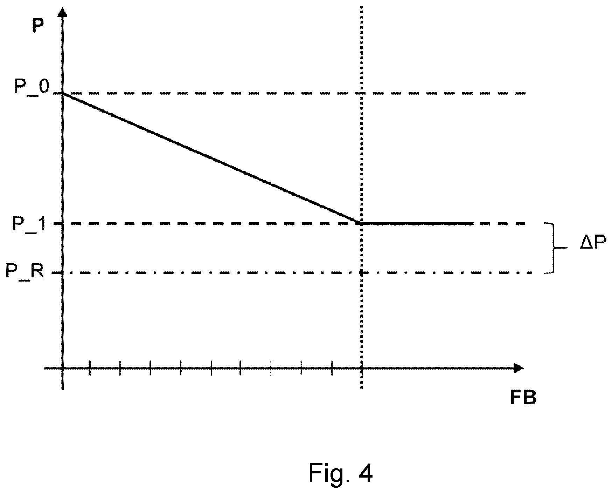

Die Steuer- oder Regelvorrichtung 29 kann im Zusammenwirken mit dem Temperatursensor TS und einer Heizvorrichtung H1 die Innentemperatur des Behälters 71 auf eine vorbestimmte Temperatur regeln. Die vorbestimmte Temperatur kann einer Temperatur entsprechen, bei welcher während der Produktion des Fördermittels, ein Referenzwert P_R (siehe Fig. 4) bestimmt wurde und einen Wert in Grad Celsius (C°) haben.

Die medizinische Behandlungsvorrichtung kann weitere Ventile, beispielsweise die Ventile V03, V05, V07 oder V9, umfassen. Mittels dieser Ventile kann der Behälter 71 bei Bedarf, beispielsweise zur Überprüfung eines anderen Fördermittels F1, F2 als dem Fördermittel F3 des oben beschriebenen Beispiels, erweitert und/oder verlegt werden. Ein Erweitern ist derart zu verstehen, dass der Behälter 71 mehr Leitungsabschnitte des Behandlungssystems 100 umfasst als im oben beschriebenen Beispiel. Ein Verlegen ist derart zu verstehen, dass der Behälter 71 andere Leitungsabschnitte der Behandlungsvorrichtung 100 umfasst als im oben beschriebenen Beispiel, wie z. B in Fig 1b dargestellt.

**Fig. 1a** zeigt den Behälter aus Fig. 1. Nicht am Zustandekommen des abgeschlossenen Behälters 71 beteiligte Bauteile der Fig. 1 sind zur besseren Übersicht in Fig. 1a nicht erneut gezeigt.

Um ihn zu befüllen, wird der Behälter 71 in manchen Ausführungsformen mittels einer Flüssigkeitsquelle 73 (siehe Fig. 1a) über Leitungen der Hydraulik gefüllt. Die Flüssigkeitsquelle 73 kann eine Quelle für Wasser, insbesondere ein Wasserhahn oder eine Wasserleitung, optional mit anschließenden Vorrichtungen zum Reinigen oder Entgasen sein oder umfassen. In anderen Ausführungsformen kann das Auffüllen optional aus einer externen abgeschlossenen Quelle, beispielsweise einem Flüssigkeitsbeutel, erfolgen. Das erfindungsgemäße Verfahren kann das Befüllen umfassen oder bei Vorliegen eines befüllten Behälters 71 erst beginnen.

Durch das Befüllen wird ein Anfangsdruck P_0 innerhalb des Behälters 71 definiert, der über die Verwendung der Flüssigkeitsquelle 73, alternativ oder ergänzend beispielsweise auch über das Ventil V13, insbesondere wenn die Füllung über eine Pumpe undefiniert erfolgt ist, geregelt bzw. eingestellt werden kann. Hierbei sind die anderen Ventile, die den Behälter 71 begrenzen, optional geschlossen.

Optional kann hierfür jedes an den Behälter 71 angeschlossene Ventil zur Füllung geöffnet und wieder geschlossen werden, hierbei sind die jeweils anderen Ventile des Behälters 71 vorzugsweise geschlossen.

Fig. 1 und Fig. 1a sind schwerpunktmäßig dem Fördermittel F3 gewidmet. Dies ist rein exemplarisch. Was zu F3 hierin ausgeführt ist, kann analog auch für F1 und/oder F2 gelten (siehe Fig. 1b).

**Fig. 1b** zeigt in dem Leitungsdiagramm des Dialysierflüssigkeitssystems 10 aus Fig. 1 einen alternativen Behälter 71', der zur Überprüfung der Fördermittel F1, F2 geeignet ist.

Hierzu sind die Ventile VB2, VS2 und V09 geschlossen. Bei Bedarf werden die Ventile VB1 und VS1 den Zugang zum Drucksensor DS1 freigeben.

Das oben zum Behälter 71 Ausgeführte trifft analog uneingeschränkt auch auf Behälter 71' zu.

Die Flüssigkeit zum Befüllen des Behälters 71' kann aus der oben links gezeigten Flüssigkeitsquelle 73 stammen.

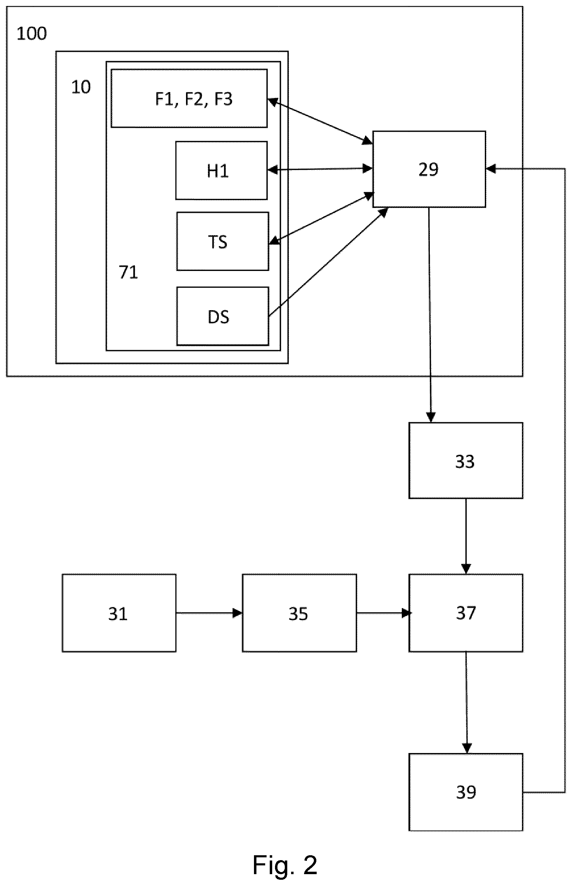

**Fig. 2** zeigt schematisch vereinfacht den Ablauf des erfindungsgemäßen Verfahrens anhand der hierfür verwendeten Vorrichtungen.

Links oben in Fig. 2 ist schematisch stark vereinfacht eine exemplarische medizinische Behandlungsvorrichtung 100 mit einem Dialysierflüssigkeitssystem 10 gezeigt. Die Steuer- oder Regelvorrichtung 29 kann konfiguriert sein, um in Zusammenwirken mit dem Temperatursensor TS und der Heizvorrichtung H1 eine vorbestimmte Innentemperatur des Behälters 71, 71' zu bewirken. Mittels des Ansteuerns von Ventilen (in Fig. 2 nicht gezeigt) oder Fördermitteln F1, F2, F3 durch die Steuer- oder Regelvorrichtung 29 kann im Inneren des Behälters 71, 71' ein vorbestimmter Anfangsdruck P_0 eingestellt werden, dies kann ggf. mittels des Drucksensors DS kontrolliert werden.

Zu seiner Überprüfung wird das Fördermittel F3, hier die Ultrafiltrationspumpe, mittels der Steuer- oder Regelvorrichtung 29 veranlasst, eine vorbestimmte Anzahl an Förderbewegungen FB (siehe Fig. 4) auszuführen, dies können, je nach Ausführungsform des Fördermittels, Hübe, Rotationen oder Schläge sein, beispielsweise fünf Hübe.

Nach Abschluss der vorbestimmten Anzahl an Förderbewegungen FB wird mittels des Druckmessers DS der im Inneren des Behälters 71, 71' herrschende Druck gemessen. Er kann, insbesondere mittels der Steuer- oder Regelvorrichtung 29, an die optional vorhandene zweite Speichervorrichtung 33 zur Speicherung darin übermittelt werden.

Der dem Fördermittel F3 entsprechende, in der ersten Speichervorrichtung 31 gespeicherte Referenzwert P_R, der ein Referenzdruck oder eine Referenzdruckdifferenz sein kann, wird von der Auslesevorrichtung 35 ausgelesen.

Die Ermittlungsvorrichtung 37 kann konfiguriert sein, um eine Druckdifferenz ΔP zwischen dem gemessenen Enddruck P_1 und gespeichertem Referenzdruck P_R zu ermitteln. Alternativ kann vorgesehen sein, dass eine Druckdifferenz ΔP ermittelt wird zwischen einer Differenz zwischen Ausgangsdruck P_0 und Enddruck P_1 einerseits und dem gespeicherten Referenzwert P_R, der in diesem Fall eine Referenzdruckdifferenz ist, andererseits.

Eine Auswertevorrichtung 39 kann konfiguriert sein, um die ermittelte Druckdifferenz ΔP auszuwerten. Es kann vorgesehen sein, das Ergebnis der Auswertung an die medizinische Behandlungsvorrichtung 100, insbesondere an deren Steuer- oder Regelvorrichtung 29, zu übermitteln.

Die medizinische Behandlungsvorrichtung 100 bzw. die Steuer- oder Regelvorrichtung 29 kann insbesondere vorgesehen sein, eine optionale Alarmvorrichtung (in Fig. 2 nicht gezeigt) zu aktivieren, wenn der Betrag der ermittelten Druckdifferenz ΔP z. B. außerhalb vorbestimmter Grenzen liegt oder auf sonstige Weise einem vorbestimmten Kriterium nicht genügt.

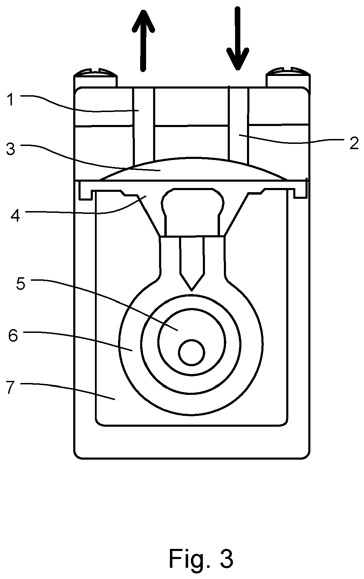

**Fig. 3** zeigt schematisch vereinfacht eine Exzenter-Membranpumpe als exemplarische Ausführungsform des Fördermittels F1, F2 oder F3 in schematischer Darstellung.

Eine elastische Membran 4 wird durch einen Exzenter 5 und einen Pleuel 6 auf und ab bewegt.

Im Abwärtshub saugt sie das zu fördernde Fluid über ein Einlassventil 2 an. Im Aufwärtshub drückt die Membran 4 das Fluid über ein Auslassventil 1 aus dem Pumpenkopf heraus. Ein Abwärtshub, ein Aufwärtshub, oder eine Kombination aus genau einem Abwärtshub mit genau einem sich anschließenden Aufwärtshub können je einer Förderbewegung FB entsprechen.

Ein Förderraum 3 ist von einem Pumpenantrieb 7 durch die Membran 4 hermetisch getrennt.

Erfindungsgemäß ist der im Förderraum 3 erzeugte Druck ein Negativdruck (Unterdruck), mittels welchem vorteilhafterweise ein Abriss der Membran 4 des Fördermittels erkannt werden kann, da bei Unterdruck vermieden wird, dass sich die Membran 4 an den Stößel legt. Würde im Förderraum 3 ein Überdruck herrschen, würde dieser die Membran 4 immer gegen den Stößel zwingen und ein Abriss könnte in einem solchen Fall unentdeckt bleiben.

**Fig. 4** zeigt einen exemplarischen Ablauf des erfindungsgemäßen Verfahrens in einem Druck [P] über einer Anzahl von Förderbewegungen [FB]-Diagramm, wobei exemplarisch auf die Anordnung der Fig. 1 und der Fig. 1a verwiesen wird.

In Fig. 4 gilt:
- P_0: ist der vorbestimmte Anfangsdruck, ein Absolutdruck; P_0 kann mittels einer Druckmessung mittels eines Absolutdruckmessers gemessen werden, er kann berechnet werden oder aus weiteren Umständen bekannt sein;
- P_1: ist der nach Beenden der Förderbewegung des betrachteten Fördermittels im Behälter 71 herrschende Druck und wird hierin als Enddruck bezeichnet;
- P_R: ist ein negativer Referenzwert für den Druck (Unterdruck) im Behälter 71, also ein negativer Druckwert, an dem der Enddruck gemessen wird, nach einer vorbestimmten Anzahl von Förderbewegungen FB des Fördermittels oder der Druckdifferenz zwischen dem Druck vor der Förderbewegung einerseits und dem Druck nach der Förderbewegung andererseits. Der Referenzwert wird vorteilhaft werkseitig ermittelt unter wenigstens einem definierten Zustand des Behälters oder wenigstens einer definierten Rahmenbedingung. Eine Rahmenbedingung kann beispielsweise eine Innentemperatur des Behälters 71 sein, wobei hier auch vorgesehen sein kann, einen engen Temperaturbereich als zulässig zu definieren.

Das Verfahren beginnt nachdem der Anfangsdruck P_0 (oder: Ausgangsdruck) im Behälter 71 bei optional einer definierten bzw. vorbestimmten Innentemperatur oder innerhalb eines vorbestimmten, engen Temperaturbereichs (siehe Fig. 1), auf den Anfangsdruck P_0 eingestellt ist, was mittels der Flüssigkeitsquelle 73 und wenigstens einem Fördermittel der medizinischen Behandlungsvorrichtung 100 erfolgen und ggf. mittels des Druckmessers DS bzw. des Temperaturmessers TS überprüft werden kann.

Das Fördermittel F3 fördert, hier exemplarisch in zehn Hüben, Flüssigkeit aus dem Behälter 71 heraus, weshalb der Druckmesser DS oder ein anderer Drucksensor nach Beendigung des Herausförderns mittels des Fördermittels F3, d. h. nach zehn Hüben, einen erfolgten Abfall des im Behälter 71 herrschenden Drucks auf P_1, den Enddruck, feststellt.

In dem gezeigten Beispiel besteht eine Druckdifferenz ΔP zwischen dem Enddruck P_1 und einem bereits hinterlegten Referenzwert P_R. In diesem Beispiel steht der Referenzwert P_R für einen Druck, der (werksseitig) nach exemplarisch 10 Hüben (vor)bestimmt wurde.

Der ermittelte Enddruck P_1 liegt oberhalb des deutlich negativeren Referenzwerts P_R, hier exemplarisch ein Unterdruck, er könnte in anderen Ausführungsformen auch ein Überdruck sein. Für den Fall, dass der Betrag der Druckdifferenz ΔP einen bestimmten Wert übersteigt, einen zulässigen Wertebereich verlässt oder ein anderes Kriterium verletzt, kann ein Alarm vorgesehen sein. Ein solcher Alarm kann akustisch oder optisch erfolgen oder könnte in manchen Ausführungsformen bereits den Hinweis umfassen, dass das Fördermittel F3 hinsichtlich seiner Funktionsfähigkeit zumindest eingeschränkt ist.

Erfolgt kein Alarm, wird der erreichte Enddruck P_1 oder die ermittelte Druckdifferenz ΔP in der zweiten Speichervorrichtung 33 (siehe Fig. 2) gespeichert.

Es kann vorgesehen sein, aus mehreren Enddrücken P_1 oder Druckdifferenzen ΔP im zeitlichen Verlauf eine Verschleißkurve zu errechnen und ggf. zu speichern. Die Verschleißkurve kann so hinterlegt sein, dass sie jederzeit von einem Servicetechniker ausgelesen bzw. angezeigt werden kann.

### Bezugszeichenliste

- 100: medizinische Behandlungsvorrichtung

- 1: Auslassventil
- 2: Einlassventil
- 3: Förderraum
- 4: Membran
- 5: Exzenter
- 6: Pleuel
- 7: Pumpenantrieb

- 10: Dialysierflüssigkeitssystem
- 29: Steuer- oder Regelvorrichtung
- 31: erste Speichervorrichtung
- 33: zweite Speichervorrichtung
- 35: Auslesevorrichtung
- 37: Ermittlungsvorrichtung
- 39: Auswertungsvorrichtung

- 71: Behälter
- 71': Behälter
- 73: Flüssigkeitsquelle

- F1: erstes Fördermittel, hier exemplarisch: Bicarbonatpumpe
- F2: zweites Fördermittel, hier exemplarisch: Natriumpumpe
- F3: drittes Fördermittel, hier exemplarisch: Ultrafiltrationspumpe

- FB: Förderbewegungen
- DS: Druckmesser, Druckmesseinrichtung
- DS1: Druckmesser
- TS: Temperaturmesser, Temperatursensor

- C1: erster Konzentratbehälter
- C2: zweiter Konzentratbehälter

- H1: Heizvorrichtung

- P_0: Anfangsdruck oder Ausgangsdruck
- P_1: Enddruck
- P_R: Referenzwert

- ΔP: (Druck-)Differenz

- t: Zeit

- V03: Ventil
- V05: Ventil
- V07: Ventil
- V09: Ventil
- V11: Ventil
- V13: Ventil
- V15: Ventil
- V17: Ventil
- V25: Ventil
- VB1, VB2: Ventile
- VS1, VS2: Ventile

## Patentansprüche

1. Verfahren zum Überprüfen der Funktionsfähigkeit eines Fördermittels (F1, F2, F3) einer medizinischen Behandlungsvorrichtung (100) zur extrakorporalen Blutbehandlung,
mit den Schritten
- Bereitstellen eines mit Flüssigkeit befüllten Behälters (71, 71'), der in Fluidverbindung oder Förderverbindung mit dem Fördermittel (F1, F2, F3) steht;
- Einstellen eines vorbestimmten Anfangsdrucks (P_0) im Behälter (71, 71');
- Ansteuern des Fördermittels (F1, F2, F3) zum Durchführen einer vorbestimmten Anzahl an Förderbewegungen (FB);
- Messen des im Behälter (71, 71') herrschenden Drucks nach Abschluss der vorbestimmten Anzahl an Förderbewegungen (FB) des Fördermittels (F1, F2, F3) mittels Druckmessung und Festlegen des gemessenen Drucks als Enddruck (P_1);
- Auslesen eines gespeicherten Referenzwerts (P_R), welcher ein Referenzdruck oder eine Referenzdruckdifferenz ist, aus einer ersten Speichervorrichtung (31);
- Ermitteln einer Druckdifferenz (ΔP) zwischen gemessenem Enddruck (P_1) und gespeichertem Referenzwert (P_R), oder Ermitteln einer Druckdifferenz (ΔP) zwischen einer Differenz zwischen Anfangsdruck (P_0) und Enddruck (P_1) einerseits und dem gespeicherten Referenzwert (P_R) andererseits; und
- Auswerten der ermittelten Druckdifferenz (ΔP);
**gekennzeichnet dadurch, dass** das Verfahren weiter die Schritte
- Einstellen einer vorbestimmten Temperatur innerhalb des Behälters (71, 71') zum Zwecke des Erreichens eines vorbestimmten Zustands während der Druckmessung und/oder Messen der während der Ausübung dieses Verfahrens im Behälter (71, 71') herrschenden Temperatur;
und/oder die Schritte
- Speichern des Enddrucks (P_1) oder der ermittelten Druckdifferenz (ΔP) in einer zweiten Speichervorrichtung (33).
- Ermitteln einer Verschleißkurve aus den gespeicherten Werten;
umfasst.

2. Verfahren nach Anspruch 1, wobei der Behälter (71, 71') Abschnitte des Altwasserzweigs eines Bilanziersystems aufweist oder hieraus besteht oder ein Abschnitt in einer Flüssigkeitsleitung eines Dialysierflüssigkeitssystems (10) ist oder umfasst.

3. Verfahren nach einem der vorangegangenen Ansprüche, wobei das Fördermittel (F1, F2, F3) eine Ultrafiltrationspumpe, eine Bicarbonatpumpe oder eine Natriumpumpe ist.

4. Verfahren nach einem der vorangegangenen Ansprüche, wobei wenigstens ein Fördermittel (F1, F2, F3) eine Verdrängerpumpe, insbesondere eine Membranpumpe, Exzenter-Membranpumpe, Schlauchpumpe, Rollenpumpe oder Kolbenpumpe ist.

5. Steuer- oder Regelvorrichtung (29), konfiguriert, um im Zusammenwirken mit einer medizinischen Behandlungsvorrichtung (100) das Verfahren gemäß einem der vorangegangenen Ansprüche zu veranlassen oder durchzuführen.

6. Medizinische Behandlungsvorrichtung (100) aufweisend oder verbunden mit jeweils wenigstens
- einem Flüssigkeitssystem, insbesondere einem Dialysierflüssigkeitssystem (10) mit einem Behälter (71, 71') für Flüssigkeit;
- einer Druckmesseinrichtung (DS) zum Messen eines im Behälter (71, 71') herrschenden Drucks;
- einer ersten Speichervorrichtung (31), in welcher ein Referenzwert (P_R), welcher ein Referenzdruck oder eine Referenzdruckdifferenz ist, gespeichert ist;
- einer Auslesevorrichtung (35) konfiguriert zum Auslesen des gespeicherten Referenzwerts (P_R);
- einer Ermittlungsvorrichtung (37) konfiguriert zum Ermitteln einer Druckdifferenz (ΔP) zwischen gemessenem Enddruck (P_1) und gespeichertem Referenzwert (P_R), oder Ermitteln einer Druckdifferenz (ΔP) zwischen einer Differenz zwischen Anfangsdruck (P_0) und Enddruck (P_1) einerseits und dem gespeicherten Referenzwert (P_R) andererseits; und
- einer Auswertevorrichtung (39) konfiguriert zum Auswerten der ermittelten Druckdifferenz (ΔP);
- wenigstens ein Fördermittel (F1, F2, F3) welches mit dem Behälter (71, 71') in Fluidverbindung oder Förderverbindung steht;
- einer Steuer- oder Regelvorrichtung (29), insbesondere gemäß Anspruch 5, konfiguriert zum Veranlassen der Durchführung eines Verfahrens nach einem der Ansprüche 1 bis 4.

7. Medizinische Behandlungsvorrichtung (100) nach Anspruch 6, mit einer Heizvorrichtung (H1), konfiguriert zum Aufheizen der Flüssigkeit, mit welcher der Behälter (71, 71') gefüllt wird, auf eine vorbestimmte Temperatur zum Zwecke des Erreichens eines vorbestimmten Zustands im Behälter (71, 71') während der auszuführenden Druckmessung.

8. Medizinische Behandlungsvorrichtung (100) nach einem der Ansprüche 6 bis 7, mit einer zweiten Speichervorrichtung (33) konfiguriert zum Speichern des Enddrucks (P_1) oder der ermittelten Druckdifferenz (ΔP) in der zweiten Speichervorrichtung (33).

9. Medizinische Behandlungsvorrichtung (100) nach einem der Ansprüche 6 bis 8, mit einer Vorrichtung, konfiguriert zum Ermitteln, z. B. Errechnen, einer Verschleißkurve aus den gespeicherten Werten.

10. Medizinische Behandlungsvorrichtung (100) nach einem der Ansprüche 6 bis 9, wobei der Behälter (71, 71') Abschnitte des Altwasserzweigs eines Bilanziersystems aufweist oder hieraus besteht.

11. Medizinische Behandlungsvorrichtung (100) nach einem der Ansprüche 6 bis 10, wobei der Behälter (71, 71') ein Abschnitt in einer Flüssigkeitsleitung eines Flüssigkeitssystems, insbesondere ein Dialysierflüssigkeitssystems (10), ist oder umfasst.

12. Medizinische Behandlungsvorrichtung (100) nach einem der Ansprüche 6 bis 11, wobei das Fördermittel (F1, F2, F3) eine Ultrafiltrationspumpe, eine Bicarbonatpumpe oder eine Natriumpumpe ist und/oder wobei das Fördermittel (F1, F2, F3) eine Verdrängerpumpe, insbesondere eine Membranpumpe, Exzenter-Membranpumpe, Schlauchpumpe, Rollenpumpe oder Kolbenpumpe ist.

13. Medizinische Behandlungsvorrichtung (100) nach einem der Ansprüche 6 bis 12, ausgestaltet als Blutbehandlungsvorrichtung, insbesondere als Hämodialysevorrichtung, Hämofiltrationsvorrichtung, Hämodiafiltrationsvorrichtung oder als eine Vorrichtung zum Durchführen eines Separationsverfahrens.

14. Digitales Speichermedium, insbesondere in Form einer Diskette, CD oder DVD oder EPROM, mit elektronisch auslesbaren Steuersignalen, konfiguriert, um eine Steuer- und/oder Regelvorrichtung zu einer erfindungsgemäßen Steuer- und/oder Regelvorrichtung (29) zu konfigurieren und/oder um eine medizinische Behandlungsvorrichtung zu einer erfindungsgemäßen medizinischen Behandlungsvorrichtung (100) zu konfigurieren, mittels der jeweils das Verfahren gemäß einem der Ansprüche 1 bis 4 veranlasst oder ausgeführt werden kann.

15. Computerprogramm-Produkt mit einem auf einem maschinenlesbaren Träger gespeicherten Programmcode, um eine Steuer- und/oder Regelvorrichtung zu einer erfindungsgemäßen Steuer- und/oder Regelvorrichtung (29) zu konfigurieren und/oder um eine medizinische Behandlungsvorrichtung zu einer erfindungsgemäßen medizinischen Behandlungsvorrichtung (100) zu konfigurieren, mittels der jeweils das Verfahren gemäß einem der Ansprüche 1 bis 4 veranlasst oder ausgeführt werden kann.

## Claims

1. A method for checking the functionality of a conveying device (F1, F2, F3) of a medical treatment apparatus (100) for the extracorporeal blood treatment,
encompassing the steps of:
- providing a container (71, 71') filled with liquid, which container (71, 71') is in fluid communication or conveying communication with the conveying device (F1, F2, F3);
- setting a predetermined initial pressure (P_0) inside the container (71, 71');
- controlling the conveying device (F1, F2, F3) in order to perform a predetermined number of conveying movements (FB);
- measuring the pressure prevailing in the container (71, 71'), using a pressure measuring, after completion of the predetermined number of conveying movements (FB) of the conveying device (F1, F2, F3) and defining the measured pressure as final pressure (P_1);
- reading out, from a first storage device (31), a stored reference value (P_R), which is a reference pressure or a reference pressure difference;
- determining a pressure difference (ΔP) between the measured final pressure (P_1) and the stored reference value (P_R), or determining a pressure difference (ΔP) between a difference between the initial pressure (P_0) and the final pressure (P_1) on the one hand and the stored reference value (P_R) on the other hand; and
- evaluating the determined pressure difference (ΔP);
**characterized in that** the method further comprises the step of:
- setting a predetermined temperature inside the container (71, 71') for the purpose of reaching or achieving a predetermined state while measuring the pressure and/or measuring the temperature prevailing, during the execution of this method, in the container (71, 71');
and/or the steps of:
- storing the final pressure (P_1) or the determined pressure difference (ΔP) in a second storage device (33),
- determining a wear curve from the stored values.

2. The method according to claim 1, wherein the container (71, 71') comprises or consists of sections of the used water branch of a balancing system, or is or comprises a section in a liquid line of a dialysis liquid system (10).

3. The method according to any one of the preceding claims, wherein the conveying device (F1, F2, F3) is an ultrafiltration pump, a bicarbonate pump, or a sodium pump.

4. The method according to any one of the preceding claims, wherein at least one conveying device (F1, F2, F3) is a displacement pump, in particular a membrane pump, an eccentric membrane pump, a tube pump, a roller pump, or a piston pump.

5. A control device or closed-loop control device (29) configured to initiate or carry out, in interaction with a medical treatment apparatus (100), the method according to any one of the preceding claims.

6. A medical treatment apparatus (100) comprising or connected to at least:
- a liquid system, in particular a dialysis liquid system (10) with a container (71, 71') for liquid;
- a pressure measuring device (DS) for measuring a pressure prevailing in the container (71, 71');
- a first storage device (31) in which a reference value (P_R), which is a reference pressure or a reference pressure difference, is stored;
- a read-out device (35) configured to read out the stored reference value (P_R);
- a determining device (37) for determining a pressure difference (ΔP) between the measured final pressure (P_1) and the stored reference value (P_R), or for determining a pressure difference (ΔP) between a difference between the initial pressure (P_0) and the final pressure (P_1) on the one hand and the stored reference value (P_R) on the other hand;
- an evaluation device (39) configured to evaluate the determined pressure difference (ΔP);
- at least one conveying device (F1, F2, F3) which is in fluid communication or conveying communication with the container (71, 71');
- a control device or closed-loop control device (29), in particular according to claim 5, configured to initiate the execution of a method according to any one of claims 1 to 4.

7. The medical treatment apparatus (100) according to claim 6, comprising a heating device (H1) configured to heat the liquid with which the container (71, 71') is being filled or will be filled to a predetermined temperature for the purpose of achieving a predetermined state in the container (71, 71') during the pressure measuring which is to be carried out.

8. The medical treatment apparatus (100) according to any one of claims 6 to 7, with a second storage device (33) configured to store the final pressure (P_1) or the determined pressure difference (ΔP) in the second storage device (33).

9. The medical treatment apparatus (100) according to any one of claims 6 to 8, with a device configured to determine, e.g. compute, a wear curve from the stored values.

10. The medical treatment apparatus (100) according to any one of claims 6 to 9, wherein the container (71, 71') comprises or consists of sections of the used water branch of a balancing system.

11. The medical treatment apparatus (100) according to any one of claims 6 to 10, wherein the container (71, 71') is or encompasses a section in a liquid line of a liquid system, in particular a dialysis liquid system (10).

12. The medical treatment apparatus (100) according to any one of claims 6 to 11, wherein the conveying device (F1, F2, F3) is an ultrafiltration pump, a bicarbonate pump or a sodium pump and/or wherein the conveying device (F1, F2, F3) is a displacement pump, in particular a membrane pump, an eccentric membrane pump, tube pump, a roller pump or a piston pump.

13. The medical treatment apparatus (100) according to any one of claims 6 to 12, designed as a blood treatment apparatus, in particular as a hemodialysis apparatus, a hemofiltration apparatus, a hemodiafiltration apparatus or as an apparatus for carrying out a separation method.

14. A digital storage medium, in particular in the form of a floppy disk, CD or DVD or an EPROM, with electronically readable control signals, configured for configuring a control device or closed-loop control device to a control device or closed-loop control device (29) according to the present invention and/or for configuring a medical treatment apparatus to a medical treatment apparatus (100) according to the present invention, by which the method according to any one of claims 1 to 4 may respectively be initiated or executed.

15. A computer program product with a program code stored on a machine-readable storage medium configured for configuring a control device or closed-loop control device into a control device or closed-loop control device (29) according to the present invention and/or for configuring a medical treatment apparatus into a medical treatment apparatus (100) according to the present invention, by which the method according to any one of claims 1 to 4 may respectively be initiated or executed.

## Revendications

1. Un procédé de vérification du bon fonctionnement d'un dispositif d'acheminement (F1, F2, F3) d'un appareil de traitement médical (100) destiné au traitement extracorporel du sang,
comprenant les étapes suivantes :
- fournir un réservoir (71, 71') rempli de liquide, qui est communication fluidique ou en communication de transport avec le dispositif d'acheminement (F1, F2, F3);
- régler une pression initiale prédéterminée (P_0) à l'intérieur du réservoir (71, 71');
- commander le dispositif d'acheminement (F1, F2, F3) afin d'effectuer un nombre prédéterminé de mouvements d'acheminement (FB);
- mesurer la pression régnant dans le réservoir (71, 71'), au moyen d'une mesure de pression, après l'achèvement du nombre prédéterminé de mouvements (FB) du dispositif d'acheminement (F1, F2, F3) et définir la pression mesurée comme pression finale (P_1);
- lire, depuis un premier dispositif d'enregistrement (31), une valeur de référence enregistrée (P_R), qui est une pression de référence ou une différence de pression de référence,
- déterminer une différence de pression (ΔP) entre la pression finale mesurée (P_1) et la valeur de référence enregistrée (P_R), ou déterminer une différence de pression (ΔP) entre une différence entre, d'une part, la pression initiale (P_0) et la pression finale (P_1) et, d'autre part, la valeur de référence enregistrée (P_R) ; et
- évaluer la différence de pression déterminée (ΔP);
**caractérisé en ce que** le procédé comprend en outre l'étape suivante :
- régler une température prédéterminée à l'intérieur du réservoir (71, 71') dans le but d'atteindre un état prédéterminé lors de la mesure de la pression et/ou mesurer la température régnant dans le réservoir (71, 71') pendant l'exécution de ce procédé;
et/ou les étapes suivantes :
- enregistrer la pression finale (P_1) ou la différence de pression déterminée (ΔP) dans un second dispositif d'enregistrement (33);
- déterminer une courbe d'usure à partir des valeurs enregistrées;

2. Le procédé selon la première revendication, où le réservoir (71, 71') comporte ou est constitué de sections de la branche des eaux usées d'un système d'équilibrage, ou est ou comporte une section dans une conduite fluidique d'un système fluidique de dialyse (10).

3. Le procédé selon l'une quelconque des revendications précédentes, où le dispositif d'acheminement (F1, F2, F3) est une pompe à ultrafiltration, une pompe à bicarbonate ou une pompe à sodium.

4. Le procédé selon l'une quelconque des revendications précédentes, où au moins un dispositif d'acheminement (F1, F2, F3) est une pompe volumétrique, notamment une pompe à membrane, une pompe à membrane excentrique, une pompe péristaltique, une pompe à rouleaux ou une pompe à piston.

5. Un dispositif de commande ou de régulation (29) configuré pour déclencher ou exécuter, en coopération avec un appareil de traitement médical (100), le procédé selon l'une quelconque des revendications précédentes.

6. Un appareil de traitement médical (100) comprenant ou étant relié respectivement à au moins :
- un système fluidique, notamment un système fluidique de dialyse (10) avec un réservoir (71, 71') pour le liquide;
- un dispositif de mesure de pression (DS) pour mesurer une pression régnant dans le réservoir (71, 71');
- un premier dispositif d'enregistrement (31) dans lequel est enregistrée une valeur de référence (P_R), qui est une pression de référence ou une différence de pression de référence;
- un dispositif de lecture (35) configuré pour lire la valeur de référence enregistrée (P_R);
- un dispositif de détermination (37) pour déterminer une différence de pression (ΔP) entre la pression finale mesurée (P_1) et la valeur de référence enregistrée (P_R), ou pour déterminer une différence de pression (ΔP) entre une différence entre, d'une part, la pression initiale (P_0) et la pression finale (P_1) et, d'autre part, la valeur de référence enregistrée (P_R); et
- un dispositif d'évaluation (39) configuré pour évaluer la différence de pression déterminée (ΔP);
- au moins un dispositif d'acheminement (F1, F2, F3) qui est en communication fluidique ou en communication de transport avec le réservoir (71, 71');
- un dispositif de commande ou de régulation (29), notamment selon la revendication 5, configuré pour déclencher l'exécution d'un procédé selon l'une quelconque des revendications 1 à 4.

7. L'appareil de traitement médical (100) selon la revendication 6, comprenant un dispositif de chauffage (H1) configuré pour réchauffer le liquide dont le réservoir (71, 71') est rempli à une température prédéterminée dans le but d'atteindre un état prédéterminé dans le réservoir (71, 71') lors de la mesure de pression à effectuer.

8. L'appareil de traitement médical (100) selon l'une quelconque des revendications 6 à 7, comprenant un second dispositif d'enregistrement (33), configuré pour enregistrer la pression finale (P_1) ou la différence de pression déterminée (ΔP) dans le second dispositif d'enregistrement (33).

9. L'appareil de traitement médical (100) selon l'une quelconque des revendications 6 à 8, comprenant un dispositif, configuré pour déterminer, par exemple pour calculer, une courbe d'usure à partir des valeurs enregistrées.

10. L'appareil de traitement médical (100) selon l'une quelconque des revendications 6 à 9, où le réservoir (71, 71') comporte ou est constitué de sections de la branche des eaux usées d'un système d'équilibrage.

11. L'appareil de traitement médical (100) selon l'une quelconque des revendications 6 à 10, où le réservoir (71, 71') est ou comporte une section dans une conduite fluidique d'un système fluidique, notamment un système fluidique de dialyse (10).

12. L'appareil de traitement médical (100) selon l'une quelconque des revendications 6 à 11, où le dispositif d'acheminement (F1, F2, F3) est une pompe à ultrafiltration, une pompe à bicarbonate ou une pompe à sodium et/ou où le dispositif d'acheminement (F1, F2, F3) est une pompe volumétrique, notamment une pompe à membrane, une pompe à membrane excentrique, une pompe péristaltique, une pompe à rouleaux ou une pompe à piston.

13. L'appareil de traitement médical (100) selon l'une quelconque des revendications 6 à 12, conçu comme un appareil de traitement du sang, notamment comme un appareil d'hémodialyse, un appareil d'hémofiltration, un appareil d'hémodiafiltration ou comme un appareil permettant d'exécuter un procédé de séparation.

14. Un support de stockage numérique, notamment sous la forme d'une disquette, d'un CD ou d'un DVD ou d'une EPROM, avec des signaux de commande lisibles électroniquement, configuré pour configurer un dispositif de commande et/ou de régulation en un dispositif de commande et/ou de régulation (29) conforme à la présente invention et/ou pour configurer un appareil de traitement médical en un appareil de traitement médical (100) conforme à la présente invention, au moyen duquel le procédé peut être déclenché ou exécuté respectivement selon l'une quelconque des revendications 1 à 4.

15. Un produit de programme informatique comprenant un code de programme enregistré sur un support lisible par machine pour configurer un dispositif de commande et/ou de régulation en un dispositif de commande et/ou de régulation (29) conforme à la présente invention et/ou pour configurer un appareil de traitement médical en un appareil de traitement médical (100) conforme à la présente invention, au moyen duquel le procédé peut être déclenché ou exécuté respectivement selon l'une des revendications 1 à 4.
